# EUROPÄISCHE PATENTSCHRIFT

(11) **EP 1 060 238 B1**
(45) Veröffentlichungstag und Bekanntmachung des Hinweises auf die Patenterteilung: **25.08.2004**
(21) Anmeldenummer: 98958772.0
(22) Anmeldetag: 22.12.1998
(51) Int. Cl.: C12M 3/00, C12N 5/00

(54) **KULTURGEFÄSS**
CULTURE DISH
RECIPIENT POUR CULTURE

(30) Priorität: 03.03.1998 CH 50698
(43) Veröffentlichungstag der Anmeldung: 20.12.2000
(73) Patentinhaber: Weidmann Plastics Technology AG, 8640 Rapperswil (CH)
(72) Erfinder: SAAD, Bashar, CH-8606 Greifensee (CH); CALLENBACH, Tilo, CH-8645 Jona (CH)
(74) Vertreter: Groner, Manfred
(86) Internationale Anmeldenummer: PCT/CH1998/000549
(87) Internationale Veröffentlichungsnummer: WO 1999/045096

(56) Entgegenhaltungen:
- EP-A- 0 300 867
- EP-A- 0 529 751
- EP-A- 0 552 412
- EP-A- 0 614 967
- EP-A- 0 751 215
- WO-A-85/02201
- WO-A-94/16058
- WO-A-95/24464
- WO-A-97/08291
- DE-C- 4 426 315
- FR-A- 2 522 014
- GB-A- 1 509 826
- US-A- 4 939 151
- US-A- 5 324 591
- US-A- 5 792 653

## Beschreibung

Die Erfindung betrifft ein Kulturgefäss aus Kunststoff für das Züchten von Zell- und Gewebekulturen nach dem Oberbegriff des Anspruchs 1. Solche Gefässe sind für die Zellzüchtung von pflanzlichen und tierischen Zellen seit langem bekannt. Früher wurden vor allem Glasflaschen und -schalen verwendet. Für den einmaligen Gebrauch haben sich Kulturgefässe vor allem aus thermoplastischen Kunststoffen, beispielsweise Polystyrol bewährt. Aufgrund der normalerweise hydrophoben Oberfläche werden solche Polystyrolgefässe durch Bestrahlung beispielsweise mit GammaStrahlen behandelt. Das Kulturgefäss kann beispielsweise eine Petrischale, eine Multischale oder beispielsweise auch eine Mikrotiter- oder Mikrotestplatte sein. Solche Kulturgefässe sind beispielsweise in der Veröffentlichung von Lindl und J. Bauer, "Zell- und Gewebekultur" 3. Auflage, Gustav Fischer Verlag (1994) beschrieben.

Bekannt sind auch Kulturgefässe, deren Wachstumsfläche durch Erhebungen vergrössert ist. Dadurch lässt sich die Zellausbeute um das 1.7 bis 2-fache erhöhen. Beispiele solcher Kulturgefässe sind in der Veröffentlichung der Firma Sigma, Nr. C 5934 aus dem Jahr 1998 zu entnehmen. Bei diesen Gefässen besteht jedoch die Schwierigkeit, dass sie die mikroskopische Untersuchung wesentlich erschweren. Sie erschweren zudem das Ernten der adhärenten Zellen.

Die EPO 552 412 A und die EPO 614 967 A zeigen Kulturgefässe, die als Flaschen ausgebildet sind und die jeweils eine Wachstumsfläche besitzen, die Rillen als Vertiefungen und Rippen als Erhöhungen aufweisen. Die Verteilung von Zell- und Gewebekulturen auf den Rippen ist hier so, dass eine mikroskopische Beobachtung nicht optimal ist. Zudem isolieren die Rillen Kulturen voneinander, was für das Wachstum ungünstig ist.

Der Erfindung liegt die Aufgabe zugrunde, ein Kulturgefäss der genannten Art zu schaffen, bei welchem die Zellen optisch besser zugänglich sind, einfacher geerntet werden können und das bei kostengünstiger Herstellung zugleich eine bessere Zellausbeute ermöglicht.

Die Aufgabe ist bei einem gattungsgemässen Kulturgefäss gemäss Anspruch 1 gelöst. Durch die Mikrostruktur der Wachstumsfläche wird die Wachstumsfläche, nicht aber das Volumen des Kulturmediums vergrössert. Eine solche Mikrostruktur erschwert das Ernten der adhärenten Zellen nicht und da die Oberfläche des Zellgefässes im wesentlichen eben ist, ist auch die mikroskopische Untersuchung nicht erschwert. Wesentlich ist auch, dass die Mikrostrukturen es den adhärenten Zellen erlauben, in einer "invivo-ähnlichen" Mikroumgebung zu wachsen. Dies dürfte erheblich zur Erhaltung des Differenzierungszustandes und zur erhöhten Zellausbeute beitragen.

Gemäss einer Weiterbildung der Erfindung weist die Wachstumsfläche Erhebungen und Vertiefungen auf, deren Höhe kleiner ist als etwa 100 Mikrometer. Diese Erhebungen und Vertiefungen werden vorzugsweise im Spritzgussverfahren hergestellt. Mit einem solchen Verfahren lassen sich solche Erhebungen mit einer Höhe von lediglich etwa 5 Nanometer herstellen. Vorzugsweise sind diese Erhebungen regelmässig angeordnet und gleich ausgebildet. Sind die Erhebungen gemäss einer Weiterbildung der Erfindung Pyramidenstümpfe, so werden regelmässige ebene obere und untere Flächen sowie geneigte Flächen geschaffen, auf welchen die Zellen wachsen können. Da diese Erhebungen sehr klein sind, liegen diese Zellen für die optische Untersuchungen dennoch im wesentlichen in der gleichen optischen Ebene. Die Erhebungen beziehungsweise Vertiefungen sind darum somit bei einem erfindungsgemässen Kulturgefäss vorzugsweise nicht statistisch und zufällig verteilt, sondern regelmässig angeordnet. Damit können auch für unterschiedliche Zellkulturen unterschiedliche Mikrostrukturen geschaffen und untersucht werden.

Ein Ausführungsbeispiel der Erfindung wird nachfolgend anhand der Zeichnung näher erläutert. Es zeigen:
- Figur 1: Schematisch ein Schnitt durch ein erfindungsgemässes Kulturgefäss mit einem Zellrasen in einer Nährlösung,
- Figur 2: eine stark vergrösserte Ansicht eines Bereichs der Wachstumsfläche, und
- Figur 3: ein Schnitt entlang der Linie III-III der Figur 2.

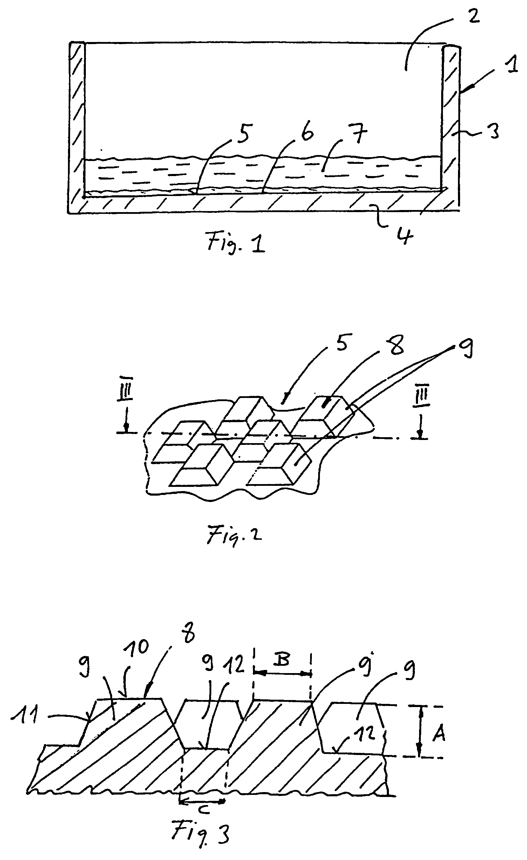

Die Figur 1 zeigt ein Kulturgefäss 1, das hier eine Petrischale ist, wobei der Deckel weggelassen ist. Das Gefäss 1 besitzt eine Gefässwandung 3 sowie einen Boden 4 und bildet einen Innenraum 2 zur Aufnahme einer Nährlösung 7 sowie einer Zellkultur 6. Die Oberseite des Bodens 4 bildet eine Wachstumsfläche 5, die wie ersichtlich im wesentlichen eben ist und auf welcher die Zellkultur 6 sich in der Nährlösung 7 befindet. Die Zellkultur 6 kann eine pflanzliche oder tierische Kultur sein. Das Gefäss 1 besteht aus einem Thermoplast, vorzugsweise aus Polystyrol und ist im Spritzgussverfahren hergestellt.

Die Wachstumsfläche 5 weist eine Mikrostruktur 8 auf, wie die Figur 2 abschnittsweise zeigt. Vorzugsweise erstreckt sich die Mikrostruktur 8 über die gesamte Wachstumsfläche 5. Denkbar ist jedoch auch eine Ausführung, bei welcher nur ein Teilbereich der Wachstumsfläche 5 eine solche Mikrostruktur 8 aufweist. Die Mikrostruktur 8 besteht ersichtlich aus einer Mehrzahl von Erhebungen 9, die vorzugsweise regelmässig angeordnet sind. Diese Erhebungen sind gemäss Figur 2 Pyramidenstümpfe. Denkbar sind aber auch Erhebungen mit anderen Formen, beispielsweise können diese Erhebungen 9 auch als Kegelstümpfe ausgebildet sein. Die Erhebungen 9 weisen obere ebene Flächen 10, vorzugsweise geneigte Seitenflächen 11 sowie untere Flächen 12 auf. Diese Flächen sind wie ersichtlich geometrisch regelmässige Flächen, vorzugsweise rechteckige Flächen.

Die in Figur 3 gezeigte Höhe A der Erhebungen 9 ist vorzugsweise gleich oder kleiner als 100 Mikrometer. Die bevorzugte Höhe A liegt im Bereich von 10 bis 60 Mikrometern. In diesem Bereich kann mit dem Mikroskop scharf gestellt werden und es bestehen vorteilhafte Wachstumsbedingungen. Die Höhe A kann aber auch wesentlich kleiner, beispielsweise 5 Nanometer, sein. Die Breite B der Flächen 10 ist vorzugsweise kleiner als 300 Mikrometer. Ebenfalls ist die Breite der Seitenflächen 11 kleiner als 300 Mikrometer. Diese Flächen 10 und 11 können sehr klein sein und beispielsweise eine Breite von etwa 5 Nanometer aufweisen. Solche Mikrostrukturen werden vorzugsweise im Spritzgussverfahren hergestellt. Die Form der Spritzgussvorrichtung besitzt dann entsprechende Bereiche mit einer solchen Mikrostruktur.

Die zu züchtenden Zellen besitzen eine sehr unterschiedliche Grösse. In der Regel ist die Mikrostruktur 8 so ausgebildet, dass die Zellen auf den Flächen 10, 11 und 12 wachsen. Die Zellen können somit in der Regel auch zwischen den Erhebungen 8 wachsen. Die Zellen können jedoch auch eine Grösse aufweisen, die mit den Erhebungen 9 vergleichbar ist. Die Zellen können auch wesentlich grösser sein als die Erhebungen 8. Für eine optimale Zelladhäsion und Zellwachstum ist das Kulturgefäss beispielsweise im Mikrowellenplasma oberflächenbehandelt.

## Patentansprüche

1. Kulturgefäss aus Kunststoff für das Züchten von Zellund Gewebekulturen (6), mit einer mikrostrukturierten Wachstumsfläche (5), die Erhebungen (9) und Vertiefungen aufweist, deren Höhe (A) kleiner ist als 110 Mikrometer, **dadurch gekennzeichnet, dass** die Erhebungen (9) jeweils eine obere ebene Fläche (10) aufweisen, dass zwischen den Erhebungen (9) ebene Flächen (12) angeordnet sind und dass benachbarte Vertiefungen miteinander verbunden sind, wobei die Erhebungen (9) Pyramiden- oder Kegelstümpfe sind.

2. Kulturgefäss nach Anspruch 2, **dadurch gekennzeichnet, dass** die Höhe (A) kleiner als 100 Mikrometer ist und vorzugsweise 10 bis 60 Mikrometer beträgt.

3. Kulturgefäss nach Anspruch 1 oder 2, **dadurch gekennzeichnet, dass** die ebene Fläche (10) eine Breite (B) aufweist, die kleiner als 300 Mikrometer ist.

4. Kulturgefäss nach einem der Ansprüche 2 bis 3, **dadurch gekennzeichnet, dass** die ebenen Flächen (10) zwischen den Erhebungen (9) jeweils eine Breite (C) oder Länge aufweisen, die kleiner als 300 Mikrometer ist.

5. Kulturgefäss nach einem der Ansprüche 1 bis 4, **dadurch gekennzeichnet, dass** die Erhebungen (9) jeweils mehrere Seitenflächen aufweisen (11), die zur Ebene der Wachsaumsfläche (5) geneigt sind oder zu dieser rechtwinklig verlaufen.

6. Kulturgefäss nach einem der Ansprüche 1 bis 5, **dadurch gekennzeichnet, dass** es im Spritzgussverfahren aus einem Thermoplast hergestellt ist.

7. Kulturgefäss nach einem der Ansprüche 1 bis 6, **dadurch gekennzeichnet, dass** es aus einem amorphen Thermoplast hergestellt ist.

8. Kulturgefäss nach Anspruch 7, **dadurch gekennzeichnet, dass** der Thermoplast Polystyrol ist.

9. Kulturgefäss nach einem der Anspüche 1 bis 8, **dadurch gekennzeichnet, dass** es für eine optimale Zelladhäsion und Zellwachstum beispielsweise im Mikrowellenplasma oberflächenbehandelt ist.

10. Kulturgefäss nach einem der Ansprüche 1 bis 9, **dadurch gekennzeichnet, dass** lediglich die Wachstumsfläche (5) eine Mikrostruktur aufweist.

11. Kulturgefäss nach einem der Ansprüche 1 bis 10, **dadurch gekennzeichnet, dass** die Wachstumsfläche (5) in regelmässiger Anordnung Erhebungen (9) aufweist und diese Erhebungen (9) geometrische und definierte Flächen (10, 11, 12) aufweisen.

12. Kulturgefäss nach einem der Ansprüche 1 bis 11, **dadurch gekennzeichnet, dass** alle oder ein wesentlicher Teil der Erhebungen (9) gleich ausgebildet sind.

13. Kulturgefäss nach einem der Ansprüche 1 bis 12, **dadurch gekennzeichnet, dass** die Wachstumsfläche (5) zwischen Erhebungen (9) gleiche und regelmässig angeordnete Flächen (12) aufweist.

14. Kulturgefäss nach Anspruch 13, **dadurch gekennzeichnet, dass** die Flächen (12) zwischen den Erhebungen (9) eben und rechteckig sind.

## Claims

1. Culture vessel made of plastic for growing cell and tissue cultures (6), with a growing surface that is provided with a microstructure (5) having elevations (9) and indentations whose height (A) is less than 110 micrometers, **characterized in that** the elevations (9) each have an upper flat surface (10), that flat surfaces (12) are arranged between the elevations (9), and that adjacent indentations are connected with one another, wherein the elevations (9) are truncated pyramids or cones.

2. Culture vessel as claimed in Claim 2 , **characterized in that** the height (A) is less than 100 micrometers and preferably 10 to 60 micrometers.

3. Culture vessel as claimed in Claim 1 or 2, **characterized in that** the flat surface (10) has a width (B) that is smaller than 300 micrometers.

4. Culture vessel as claimed in one of Claims 2 to 3, **characterized in that** the flat surfaces (10) between the elevations (9) each have a width (C) or length that is smaller than 300 micrometers.

5. Culture vessel as claimed in any one of Claims 1 to 4, **characterized in that** the elevations (9) each have several lateral surfaces (11) which are inclined in relation to the growing surface (5) or extend perpendicularly thereto.

6. Culture vessel as claimed in any one of Claims 1 to 5, **characterized in that** it is produced from a thermoplastic by means of an injection molding process.

7. Culture vessel as claimed in any one of Claims 1 to 6, **characterized in that** it is made from an amorphous thermoplastic.

8. Culture vessel as claimed in Claim 7, **characterized in that** the thermoplastic is polystyrene.

9. Culture vessel as claimed in any one of Claims 1 to 8, **characterized in that** it is surface treated for optimal cell adhesion and cell growth, for example, in a microwave plasma.

10. Culture vessel as claimed in any one of Claims 1 to 9, **characterized in that** only the growing surface (5) has a microstructure.

11. Culture vessel as claimed in any one of Claims 1 to 10, **characterized in that** the growing surface (5) has regularly arranged elevations (9) and these elevations (9) have geometric and defined surfaces (10, 11, 12).

12. Culture vessel as claimed in any one of Claims 1 to 11, **characterized in that** all or a substantial part of the elevations (9) are identical in shape.

13. Culture vessel as claimed in any one of Claims 1 to 12, **characterized in that** the growing surface (5) has identical and regularly arranged surfaces (12) between the elevations (9).

14. Culture vessel as claimed in Claim 13, **characterized in that** the surfaces (12) between the elevations (9) are flat and rectangular.

## Revendications

1. Récipient de culture en matière plastique pour l'incubation de cultures de tissus et de cellules (6), comportant une surface de croissance (5) microtexturée, qui présente des saillies (9) et des creux, dont la hauteur (A) est inférieure à 110 micromètres, **caractérisé en ce que** les saillies (9) présentent chacune une surface plane supérieure (10), **en ce que** des surfaces planes (12) sont disposées entre les saillies (9) et **en ce que** des creux voisins sont reliés entre eux, les saillies (9) étant des troncs de pyramides ou de cônes.

2. Récipient de culture selon la revendication 1, **caractérisé en ce que** la hauteur (A) est inférieure à 100 micromètres et va de préférence de 10 à 60 micromètres.

3. Récipient de culture selon la revendication 1 ou 2, **caractérisé en ce que** la surface plane (10) présente une largeur (B) qui est inférieure à 300 micromètres.

4. Récipient de culture selon la revendication 2 ou 3, **caractérisé en ce que** les surfaces planes (10) entre les saillies (9) présentent chacune une largeur (C) ou une longueur qui est inférieure à 300 micromètres.

5. Récipient de culture selon l'une quelconque des revendications 1 à 4, **caractérisé en ce que** les saillies (9) présentent chacune plusieurs faces latérales (11) qui sont inclinées par rapport au plan de la surface de croissance (5) ou sont à angle droit par rapport à celle-ci.

6. Récipient de culture selon l'une quelconque des revendications 1 à 5, **caractérisé en ce qu'**il est fabriqué par le procédé de moulage par injection à partir d'une matière thermoplastique.

7. Récipient de culture selon l'une quelconque des revendications 1 à 6, **caractérisé en ce qu'**il est fabriqué à partir d'une matière thermoplastique amorphe.

8. Récipient de culture selon la revendication 7, **caractérisé en ce que** la matière thermoplastique est le polystyrène.

9. Récipient de culture selon l'une quelconque des revendications 1 à 8, **caractérisé en ce qu'**il est traité en surface, par exemple dans le plasma à micro-ondes, pour une adhérence optimale des cellules et une croissance optimale des cellules.

10. Récipient de culture selon l'une quelconque des revendications 1 à 9, **caractérisé en ce que** seule la surface de croissance (5) présente une microtexture.

11. Récipient de culture selon l'une quelconque des revendications 1 à 10, **caractérisé en ce que** la surface de croissance (5) présente des saillies (9) disposées régulièrement et ces saillies (9) présentent des faces géométriques et définies (10, 11, 12).

12. Récipient de culture selon l'une quelconque des revendications 1 à 11, **caractérisé en ce que** la totalité ou une grande partie des saillies (9) ont la même configuration.

13. Récipient de culture selon l'une quelconque des revendications 1 à 12, **caractérisé en ce que** la surface de croissance (5) entre les saillies (9) présente des faces (12) égales et disposées régulièrement.

14. Récipient de culture selon la revendication 13, **caractérisé en ce que** les faces (12) entre les saillies (9) sont planes et rectangulaires.
